# EUROPEAN PATENT SPECIFICATION

(11) **EP 3 169 675 B1**
(45) Date of publication and mention of the grant of the patent: **19.09.2018**
(21) Application number: 15739271.3
(22) Date of filing: 17.07.2015
(51) Int. Cl.: C07D 401/12, A61K 31/5377, A61P 29/00, A61P 31/18, A61P 1/00, A61P 19/02, A61P 25/00

(54) **A QUINOLINE DERIVATIVE FOR THE TREATMENT OF INFLAMMATORY DISEASES AND AIDS**
CHINOLINDERIVAT ZUR BEHANDLUNG VON ENTZÜNDUNGSKRANKHEITEN UND AIDS
DÉRIVÉ DE QUINOLÉINE POUR LE TRAITEMENT DE MALADIES INFLAMMATOIRES ET DU SIDA

(30) Priority: 17.07.2014 EP 14306166
(43) Date of publication of application: 24.05.2017
(73) Proprietor: ABIVAX, 75008 Paris (FR); Centre National de la Recherche Scientifique, 75794 Paris Cedex 16 (FR); Institut Curie, 75248 Paris Cedex 05 (FR); Université de Montpellier, 34090 Montpellier (FR)
(72) Inventor: TAZI, Jamal, 34380 Clapiers (FR); NAJMAN, Romain, 94240 L'Hay-les-Roses (FR); MAHUTEAU, Florence, 78470 Saint Rémy les Chevreuses (FR); SCHERRER, Didier, 34170 Castelnau le Lez (FR); GARCEL, Aude, 34160 Castries (FR); CAMPOS, Noëlie, 34920 Le Cres (FR)
(74) Representative: Nony
(86) International application number: PCT/EP2015/066462
(87) International publication number: WO 2016/009066

(56) References cited:
- EP-A1- 2 465 502
- WO-A1-2004/007461

## Description

The present invention relates to a compound of formula (1) 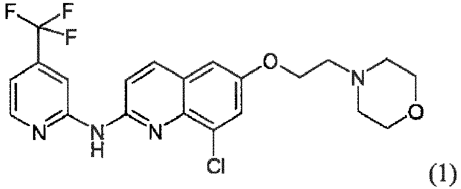 and to the pharmaceutical composition comprising it. This compound can be used in the treatment of retroviral infection, in particular AIDS or an AIDS-related condition or Human Immunodeficiency virus (HIV), and also in the treatment and/or prevention of inflammatory disease. The invention also relates to a process for preparing the compound of formula (1) and also to one intermediate compound in said process.

The scope of the invention is defined by the claims. Any references in the description to methods of treatment refer to the compounds, pharmaceutical compositions and medicaments of the present invention for use in a method for treatment of the human (or animal) body by therapy. One of the strategies to combat retroviral infections and more particularly AIDS is to use derivatives able to selectively inhibit certain splicing defects. This is in particular the aim in document WO 2010/143169.

In fact, International application WO 05023255, filed by the Applicant, disclosed the use of indole derivatives to treat diseases related to the pre-messenger RNA splicing process in the cell.

Thus it was recently shown that certain indole derivatives prove particularly effective in treating metastatic cancer and in treating AIDS (BAKKOUR et al., PLoS Pathogens, vol. 3, p. 1530-1539, 2007).

The inventors developed a novel compound that is particularly effective in treating diseases related to the splicing process, but which, in a surprising manner, have a cellular toxicity that is clearly less than the indole derivatives of the prior art. In addition, said compound is able to selectively inhibit certain splicing events.

In parallel, inflammation is a protective response by the immune system to tissue damage and infection. However, the inflammatory response, in some circumstances, can damage the body. In the acute phase, inflammation is characterized by pain, heat, redness, swelling and loss of function. There are a wide range of inflammatory conditions which affect millions of people worldwide.

MicroRNAs (miRNA), the most comprehensive noncoding group, are a class of about 22 nt noncoding RNAs that inhibit gene expression through binding to the UnTranslated Region (UTR) of target mRNA transcripts (Lai et al., Nature Genetics, vol. 30, no. 4, pp. 363-364, 2002; Bartel et al., Cell, vol. 136, no. 2, pp. 215-233, 2009). miRNA genes represent about 1-2% of the known eukaryotic genomes. Predictions suggest that each miRNA can target more than 200 transcripts and that a single mRNA can be regulated by multiple miRNAs (LINDOW, DNA Cell Biol., vol. 26(5), p. 339-351, 2007). miRNAs are generated from endogenous hairpin-shaped transcripts and act by base pairing with target mRNAs, which leads to mRNA cleavage or translational repression, depending on the degree of base-pairing. Two processing events lead to mature miRNA formation: first, the nascent miRNA transcripts (pri-miRNA) are processed into 70 nucleotides precursors (pre-miRNA) which are exported from the nucleus and are cleaved in the cytoplasm to generate short (about 22 nucleotides long) mature miRNAs (LEE, EMBO J., vol. 21, p; 4663-4670, 2002). miRNAs can be located inter- or intragenically. When intergenic, their expression is coordinated with other miRNAs as a cluster (Altuvia et al., Nucleic Acids Research, vol. 33, no. 8, pp. 2697-2706, 2005, Ozsolak et al., Genes and Development, vol. 22, no. 22, pp. 3172-3183, 2008). When intragenic, namely, positioned within a protein-coding gene (almost exclusively in introns), they are often expressed from the same strand as their host-gene (Liu et al., Cell Research, vol. 18, no. 10, pp. 985-996, 2008, Kim et al., EMBO Journal, vol. 26, no. 3, pp. 775-783, 2007) and at correlated levels (Baskerville et al., RNA, vol. 11, no. 3, pp. 241-247, 2005).

miR-124 was initially cloned in mouse. Human miR-124 precursor (or miRN-124 or miRNA-124 or micro RNA 124) was cloned from embryonic stem cells. 9 haplotypes of miR-124 precursors have been identified so far (Guo et al., PLoS ONE, 2009, 4(11):e7944), from which 3 are present in the Human, hsa-miR-124-1, hsa-miR-124-2 and hsa-miR-124-3. (Respectively **SEQ ID NO: 1, SEQ ID NO: 2** and **SEQ ID NO: 3**).

The miR-124 microRNA precursor is a small non-coding RNA molecule. The mature ∼21 nucleotide microRNAs are processed from hairpin precursor sequences by the Dicer enzyme. The mature sequences are **SEQ ID NO: 4** for miR-124-3p and **SEQ ID NO: 5** for miR-124-5p.

It has now been shown that overexpression of miR-124, deactivates inflammatory macrophages and converts them into microglia-like cells. miR-124 is believed to inhibit macrophage activation by targeting CEBPα, a transcription factor responsible for the differentiation of myeloid lineage cells. Intravenous injection of liposomes containing miR-124 markedly suppresses clinical EAE symptoms and inhibits the infiltration of encephalitogenic T cells and inflammatory macrophages into the CNS.

Indeed, Ponomarev et al. ("microRNA-124 promotes microglia quiescence and suppresses EAE by deactivating macrophages via the C/EBP-α-PU.1 pathway"; Nature Medicine (2011); 17:1: 64-71) suggests that miR-124 could play a role as a key regulator of microglia quiescence and as a modulator of monocyte and macrophage activation. Based on an Experimental Autoimmune Encephalomyelitis (EAE) model, this study suggests that the miR-124 expression pattern is modulated (either up-regulated or downregulated depending on the cell type) in mice with EAE.

WO 2010/151755 also teaches the administration of miR-124 for treating a central nervous system (CNS) inflammatory disease.

Sun et al. ("microRNA-124 mediates the cholinergic anti-inflammatory action through inhibiting the production of pro-inflammatory cytokines"; Cell Research (2013); 23:1270-1283) also teaches that miR-124 could mediate a cholinergic anti-inflammatory action through targeting of STAT3 and TACE.

WO 2004/007461 discloses 8-hydroxy quinoline derivatives for treating multiple sclerosis and inflammatory bowel disease.

On the other hand, document WO 2012/080953 discloses compounds of formula (I) 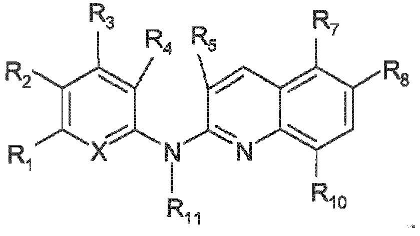 Said compounds are more particularly useful for treating AIDS. In fact, said compounds are described as being useful in the treatment of diseases resulting from changes in splicing processes, which may constitute one strategy in the treatment of AIDS.

In the framework of the present invention, the term "patient" may extend to humans or mammals, such as cats or dogs.

According to a first aspect, the invention relates to the compound of formula (1): 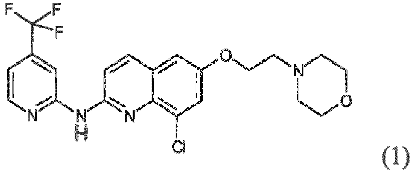 or one of its pharmaceutically acceptable salts.

Said compound may exist in the form of a base or an addition salt with an acid, particularly a pharmaceutically acceptable acid.

Suitable physiologically acceptable acid addition salts of compound of formula (1) include hydrobromide, tartrate, citrate, trifluoroacetate, ascorbate, hydrochloride, tartrate, triflate, maleate, mesylate, formate, acetate and fumarate.

The compound of formula (1) and or salts thereof may form solvates or hydrates and the invention includes all such solvates and hydrates.

The terms "hydrates" and "solvates" simply mean that the compound of formula (1) according to the invention can be in the form of a hydrate or solvate, i.e. combined or associated with one or more water or solvent molecules.

The compound of formula (1) has the following chemical name: 8-chloro-6-(2-morpholinoethoxy)-*N*-(4-(trifluoromethyl)pyridin-2-yl)quinolin-2-amine.

According to another aspect, a subject-matter of the present invention relates to a compound of formula (1) or its pharmaceutically acceptable salts, for use as a medicament.

According to another of its objects, the invention relates to a pharmaceutical composition comprising a compound of formula (1) or its pharmaceutically acceptable salts and at least one pharmaceutically acceptable excipient, and to the medicament comprising the compound of formula (1) or one of its pharmaceutically acceptable salts.

This compound can be used in the treatment of a retroviral infection, AIDS, an AIDS-related condition or HIV, and also in the treatment and/or prevention of inflammatory disease.

The compound of formula (1) which is suitable for the invention may be prepared according to Scheme I below:

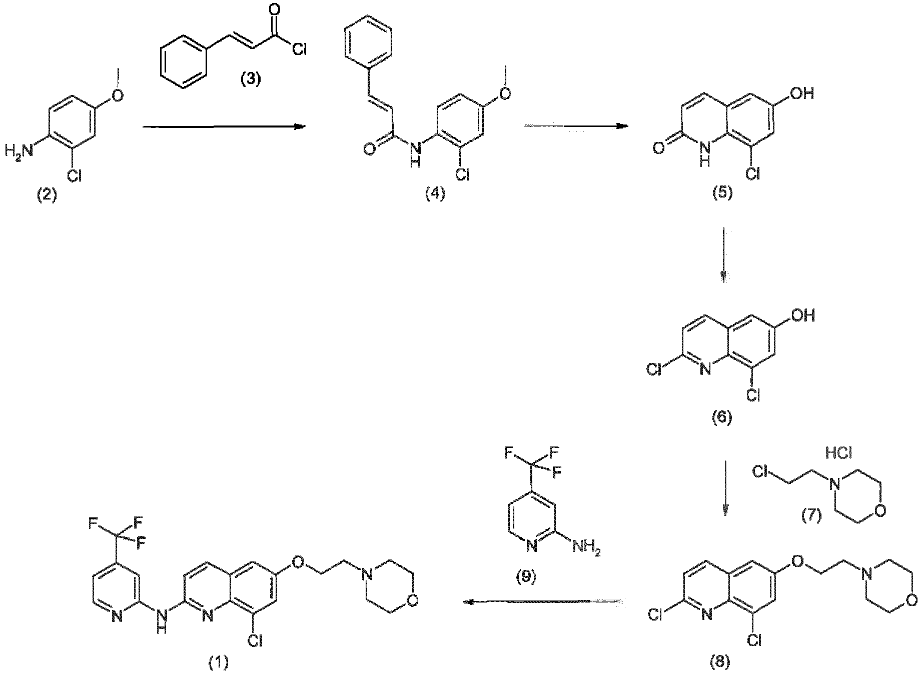

Compound (**2**), which is commercially available, can be placed in a mixture of acetone and water in the presence of an inorganic base, such as Cs₂CO₃ or K₂CO₃, in a molar ratio ranging from 1 to 4, for example 3. At 0°C, compound (**3**), which may be obtained according to preparation methods according to the common knowledge of the man skilled in the art, can then be added in a molar ratio ranging from 1 to 1.5, for example 1, with respect to compound (**2**). The reaction mixture can be allowed to warm-up to room temperature and be stirred for a time ranging from 2 hours to 24 hours, for example during 20 hours. The resulting precipitate can be filtered, rinsed with water and dried under reduced pressure in a desiccator to afford compound (**4**).

Compound (**4**) can be placed in an aprotic solvent such as chlorobenzene in the presence of aluminium trichloride in a molar ratio ranging from 5 and 10, for example 6. The reaction mixture can then be heated at a temperature ranging from 100 to 150°C, for example at 130°C, and stirred for a time ranging from 1 to 5 hours, for example during 4 hours, under an inert atmosphere of gas, for example argon. Upon cooling to room temperature, the reaction mixture can be poured onto an ice/water mixture. The resulting precipitate can be filtered, rinsed with water and dried under reduced pressure in a desiccator to afford compound (**5**).

Compound (**5**) can be placed in POCl₃ as the solvent and the reaction mixture can then be heated at a temperature ranging from 100 to 120°C, for example at 110°C, and stirred for a time ranging from 1 to 5 hours, for example during 4 hours. Upon cooling to room temperature, water can slowly be added to the reaction mixture. The resulting precipitate can then be filtered, washed with water and dried under reduced pressure in a desiccator to afford compound (**6**).

Compound (**6**) can be placed in an anhydrous polar solvent such as anhydrous *N,N*-dimethylformamide in the presence of KI in a molar ratio ranging from 1 to 2, for example 1, and in the presence of an inorganic base such as cesium carbonate in a molar ratio ranging from 1 to 2, for example 1, with respect to compound (**6**). Compound (**7**) can then be added in a molar ratio ranging from 1 to 1.5, for example 1, with respect to compound (**6**). The reaction mixture can be heated at a temperature ranging from 70 to 110°C, for example at 90°C, and stirred for a time ranging from 7 hours to 24 hours, for example 20 hours. Upon cooling to room temperature, the reaction mixture can be concentrated under reduced pressure and the resulting residue can be diluted with an organic solvent such as ethyl acetate. The organic phase can then be washed with a saturated aqueous solution of brine, dried over MgSO₄, filtered and concentrated under reduced pressure to. The organic phases can then be gathered, washed with a saturated aqueous solution of brine, dried over MgSO₄, filtered and concentrated under reduced pressure to afford compound (**8**).

Compound (**8**) can be placed in a protic solvent such as *t*-BuOH. Compound (**9**) can then be added in a molar ratio ranging from 1 to 2, for example 1, with respect to compound (**8**), in the presence of an inorganic base, such as Cs₂CO₃ or K₂CO₃, in a molar ratio ranging from 2 to 5, for example 2.8, in the presence of a diphosphine, such as Xantphos (4,5-Bis(diphenylphosphino)-9,9-dimethylxanthene) or X-Phos (2-Dicyclohexylphosphino-2',4',6'-triisopropylbiphenyl) in an amount ranging from 2 mol% to 10 mol% relative to the total amount of compound (**8**), and in the presence of a catalyst, such as Pd(OAc)₂ or Pd₂(dba)₃ in an amount ranging from 2 mol% to 10 mol% relative to the total amount of compound (**8**). The reaction mixture can be heated in a microwave reactor at a temperature ranging from 90 to 150°C, for example at 120°C, for a time ranging from 30 minutes to 100 minutes, for example 30 minutes. The reaction mixture can be concentrated under reduced pressure and the residue can be diluted with an organic solvent such as ethyl acetate. The organic phase can be washed with water, decanted, dried over magnesium sulphate, filtered and concentrated under reduced pressure to afford compound (1).

Therefore, the invention also relates to the process for preparing the compound of formula (1), comprising the step of reacting a compound of formula (8) with a compound of formula (9) as defined above, in the presence of an inorganic base such as a carbonate base (for example cesium, potassium or sodium) or a tert-butoxide base (for example cesium, potassium or sodium), in the presence of a catalyst, preferably a Palladium catalyst, and preferably in the presence of a ligand such as a phosphine ligand or a ferrocene-derived ligand, for example a bidentate phosphine ligand, optionally with heating at a temperature ranging from 90 to 150°C.

The invention also extends to the compound of formula (8), which is an intermediate compound:

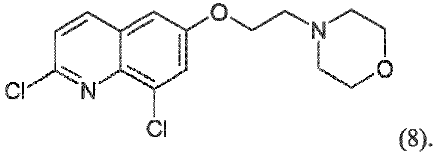

### Retroviral infection and AIDS

Viruses, in particular from the retroviral family, are one of the major causes of diseases around the world. Three subfamilies can be distinguished within the retroviral family: the oncoviruses, the lentiviruses and the spumaviruses.

The oncoviruses are thus termed because they can be associated with cancers and malignant infections. There may be mentioned, for example, leukemogenic viruses such as the avian leukemia virus (ALV), the murine leukemia virus (MULV), also called Moloney virus, the Abelson leukemia virus, the murine mammary tumor virus, the Mason-Pfizer monkey virus (or MPMV), the feline leukemia virus (FELV), human leukemia viruses such as HTLV1 (also,named HTLV-I) and HTLV2 (also named HTLV-II), the simian leukemia virus or STLV, the bovine leukemia virus or BLV, the primate type D oncoviruses, the type B oncoviruses which are inducers of mammary tumors, or oncoviruses which cause a rapid cancer (such as the Rous sarcoma virus or RSV).

Although the term oncovirus is still commonly used, other terms can also be used such as Alpharetrovirus for avian leukosis virus and Rous sarcoma virus; Betaretrovirus for mouse mammary tumor virus; Gammaretrovirus for murine leukemia virus and feline leukemia virus; Deltaretrovirus for bovine leukemia virus and human T-lymphotropic virus; and Epsilonretrovirus for Walleye dermal sarcoma virus.

The spumaviruses manifest fairly low specificity for a given cell type or a given species, and they are sometimes associated with immunosuppressive phenomena; that is the case, for example, for the simian foamy virus (or SFV), also named chimpanzee simian virus, the human foamy virus (or HFV), bovine syncytial virus (or BSV), feline syncytial virus (FSV) and the feline immunodeficiency virus.

The lentiviruses, such as Human Immunodeficiency Virus (HIV, also known as HTLV-III or LAV for lymphotrophic adenovirus and which can be distinguished within HIV-1 and HIV-2), are thus named because they are responsible for slow-progressing pathological conditions which very frequently involve immunosuppressive phenomena, including AIDS. Among the lentiviruses, the visna/maedi virus (or MVV/Visna), equine infectious anemia virus (EIAV), caprine arthritis encephalitis virus (CAEV), simian immunodeficiency virus (SIV) can also be cited. Certain indole derivative compounds such as ellipticine derivatives and aza-ellipticine derivatives are already known as intercalating molecules for correcting dysfunctions in gene expression, notably in DNA replication. They have been more specifically described for treating diseases such as cancer, leukemia or AIDS (see in particular patents FR 2 627 493, FR 2 645 861, FR 2 436 786).

According to the invention, "HIV" includes both HIV-1 and HIV-2, and preferably HIV-1.

In view of the above, the invention relates to a compound of formula (1) for use in the treatment of retroviral infection, and in particular in the treatment of AIDS, an AIDS-related condition or HIV.

Among retroviruses, the following may be cited: visna/maedi virus or MVV/visna, equine infectious anemia virus or EIAV, caprine arthritis encephalitis virus or CAEV, simian immunodeficiency virus or SIV, avian leukemia virus or ALV, murine leukemia virus also called Moloney virus or MULV, Abelson leukemia virus, murine mammary tumor virus, Mason-Pfizer monkey virus or MPMV, feline leukemia virus or FELV, human leukemia viruses HTLV-I, human leukemia viruses HTLV-II, simian leukemia virus or STLV, bovine leukemia virus or BLV, primate type D oncoviruses, type B oncoviruses, Rous sarcoma virus or RSV, simian foamy virus or SFV or chimpanzee simian virus, human foamy virus, and feline immunodeficiency virus, the human foamy virus or HFV, bovine syncytial virus or BSV, feline syncytial virus FSV, the feline immunodeficiency virus, avian leukosis virus, Walleye dermal sarcoma virus, T-cell lymphoma, acute ATL, lymphomatous ATL, chronic ATL, smoldering ATL, neurologic diseases, Tropical spastic paraparesis or HTLV-associated myelopathy, inflammatory and autoimmune diseases such as uveitis, dermatitis, pneumonitis, rheumatoid arthritis, and polymyositis hematologic and dermatologic diseases, lung diseases, brain diseases, and/or immunodeficiency.

The invention also relates to the use of a compound of formula (1) for the preparation of a composition, such as a medicament, for treating a retroviral infection and more particularly AIDS, an AIDS-related condition or HIV.

According to one aspect, the present invention relates to a method consisting in contacting a cell having a retroviral infection with one compound of formula (1).

In yet another aspect, the present invention relates to a method for treating a retroviral infection or a disease caused by the retroviral infection comprising administering an effective amount of a pharmaceutical composition to a patient in need thereof, wherein the pharmaceutical composition includes at least a compound of formula (1) and wherein in particular the retroviral infection is HIV and wherein in particular the disease caused by the retroviral infection is AIDS, an AIDS-related condition or HIV.

### Inflammatory diseases

Inflammatory diseases include a wide range of conditions including, inflammatory disease associated with an autoimmune disease, a central nervous system (CNS) inflammatory disease, a joint inflammation disease, an inflammatory digestive tract disease, and inflammatory skin. Among them, Inflammatory Bowel Disease, Rheumatoid Arthritis and Multiple Sclerosis are of particular interest.

**Inflammatory Bowel Disease (IBD)** is a complex multifactorial disease (Perš̌̌e and Cerar, 2012). It commonly refers to ulcerative colitis (UC) and Crohn's disease (CD), the two chronic conditions that involve inflammation of the intestine. IBD is common in developed countries, with up to 1 in 200 of individuals of Northern European region affected by these diseases. Patients with IBD present several clinically challenging problems for physicians. DSS-induced colitis is associated with the upregulation of different proinflammatory cytokines including TNFalpha and IFNgamma. Recently, miR-124 has been shown to be de-regulated specifically in pediatric patients with active UC, leading to increased levels of transducer and activator of transcription 3 (STAT3) expression and the transcriptional activation of its downstream targets among which proinflammatory cytokines (Koukos et al.; Gastroenterology; 145(4):842-52: 2013). However, in spite of recent advances, there remains a need for a safe, well-tolerated therapy with a rapid onset, and increased capacity for maintaining long-term remission.

**Rheumatoid arthritis (RA)** is the most frequent autoimmune disease with a prevalence of about 0.3 to 1% of the population worldwide and often associated with reduced mobility, increased social dependency and work disability. RA is a systemic inflammatory disease affecting the joint lining tissue called synovium. The rheumatoid synovial tissue is characterized by hyper-proliferation of fibroblast-like synoviocytes (FLS) in the intimal lining layer and infiltration of the sublining by macrophages, T and B cells, and other inflammatory cells that promote inflammation and destruction of bone and cartilage. The intra-articular and systemic expression of pro-inflammatory cytokines, in particular tumor necrosis factor alpha (TNFα), interleukin-1 (IL-1) and -6 (IL-6), which are primarily produced by synovial macrophages, plays a crucial role in the pathogenesis of RA, e.g. by contributing to the hyper-proliferation of RA FLS. RA patients are in general treated with a group of small molecular drugs called disease modifying antirheumatic drugs (DMARDs). DMARDs suppress the body's overactive immune and/or inflammatory systems in some way, thereby slowing down disease progression. RA patients not responding to DMARDs are treated with biological agents such as Tumor Necrosis Factor (TNF) antagonists. However, even though TNF antagonists are effective in about two-thirds of the patients, the responding patients frequently become non-responsive within five years. Therefore, alternative treatments are required. Notably, there is a particular interest for novel therapeutic approaches designed for RA patients at early stages, before the disease becomes chronic.

**Multiple sclerosis (MS)** is an inflammatory disease autoimmune, demyelinating disease of the central nervous system that destroys myelin, oligodendrocytes, and axons. MS is characterized by multiple foci of inflammation and infiltration of macrophages and encephalitogenic T cells in the central nervous system. Microglia are found throughout the central nervous system and participate in the onset and progression of CNS inflammatory responses. Microglia, when activated, are highly damaging to CNS function through their production of neurotoxins, inflammatory cells (Inflammatory Protein-10, Macrophage Inflammatory Protein-1, Macrophage Inflammatory Protein-2, C-C Chemokine Ligand 19, Monocyte Chemoattractant Protein-1, Monocyte Chemoattractant Protein-2) and immune cells that produce antibodies. Microglia direct inflammatory responses that can result in the brain and spinal cord being infiltrated with immune cells against foreign invaders as well as T-cells that destroy myelin proteins. Peripheral macrophages appear in the CNS during inflammation and these cells have a highly activated phenotype, efficiently stimulate expansion of encephalitogenic T cells, and are thought to contribute to neuronal tissue destruction.

According to the invention, an « *inflammation* » is characterized by pain, heat, redness and swelling, and can result from infection, irritation, or injury.

Thus, an « *inflammatory disease* » refers to a group of diseases and/or disorders that are caused by an excessive or dysregulated inflammation.

According to the invention, « *treating and*/*or preventing an inflammatory disease* » may either refer to the treatment and/or prevention of an inflammatory disease, or to inflammation itself, which may occur along with said inflammatory disease in an individual.

Thus, a treatment and/or prevention of an inflammatory disease also includes a treatment and/or prevention of inflammation as such.

According to the invention, *"treating and*/*or preventing"* an inflammatory disease includes treating, reducing the likelihood of developing, or delaying the occurrence of said inflammatory disease.

According to the invention, an "*individual*" may relate to a human or non-human mammal, and preferably to a human.

In a non-limitative manner, inflammatory diseases include: an inflammatory disease associated with an autoimmune disease, a central nervous system (CNS) inflammatory disease, a joint inflammation disease, an inflammatory digestive tract disease, inflammatory skin and other inflammatory diseases related to epithelial cells such as bronchitis, inflammation associated with cancer, such as colon carcinoma, inflammation associated with irritation, and inflammation associated with injury.

Thus, an inflammatory disease can be selected in the list consisting of: an inflammatory disease associated with an autoimmune disease, a central nervous system (CNS) inflammatory disease, a joint inflammation disease, an inflammatory digestive tract disease, inflammatory skin and other inflammatory diseases related to epithelial cells, inflammation associated with cancer, inflammation associated with irritation, and inflammation associated with injury.

In particular, an inflammatory disease is selected in the list consisting of: Inflammatory Bowel Disease, Rheumatoid Arthritis, Crohn's disease, Ulcerative Colitis, Multiple Sclerosis, osteoarthritis, ankylosing spondylitis, psoriasis, Sjogren's syndrom, bronchitis, and colon carcinoma.

More particularly, an inflammatory disease is selected in the list consisting of: Inflammatory Bowel Disease, Rheumatoid Arthritis, Crohn's disease, Ulcerative Colitis, Multiple Sclerosis, osteoarthritis, ankylosing spondylitis, and psoriasis.

Preferably, an inflammatory disease according to the invention includes: flammatory Bowel Disease, Crohn's disease, Ulcerative Colitis, Rheumatoid Arthritis and Multiple Sclerosis.

Even more preferably, an inflammatory disease according to the invention includes: Inflammatory Bowel Disease, Rheumatoid Arthritis and Multiple Sclerosis.

In view of the above, the invention relates to a compound of formula (1) for use in the treatment and/or prevention of an inflammatory disease, which encompasses inflammation as such, and inflammation associated with an inflammatory disease.

Thus, the invention also relates to the use of a compound of formula (1) for treating and/or preventing an inflammatory disease, which encompasses inflammation as such, and inflammation associated with an inflammatory disease.

The invention also relates to the use of a compound of formula (1) for the preparation of a composition, such as a medicament, for treating and/or preventing inflammation, which encompasses inflammation as such, and inflammation associated with an inflammatory disease.

According to one aspect, the present invention relates to a method consisting in contacting a cell having an inflammation with one compound of formula (1).

The invention also relates to a method for treating and/or preventing an inflammatory disease, which includes inflammation as such, and inflammation associated with said inflammatory disease, and which comprises a step of administering an effective amount of a compound of formula (1) to a patient in need thereof.

The following examples are provided as illustrations of the present invention.

### Example 1: Synthesis of compound (1)

2-chloro-4-methoxyaniline (**2**) (2.3 mL, 18.0 mmoles, 1 eq.) was placed in water (13 mL) in the presence of K₂CO₃ (7.5 g, 54.0 mmoles, 3 eq.). A solution of (2E)-3-phenylprop-2-enoyl chloride (**3**) (3 g, 18.0 mmoles, 1 eq.) in acetone (18 mL) was then added at 0°C. The reaction mixture was allowed to warm-up to room temperature and stirred for 20 hours. The resulting precipitate was filtered, rinsed with water and dried under reduced pressure in a desiccator to afford (2E)-*N*-(2-chloro-4-methoxyphenyl)-3-phenylprop-2-enamide (**4**) (3.6 g, 69%).
¹H NMR (300 MHz, CDCl₃) δ 8.37 (d, *J* = 9.0 Hz, 1H), 7.77 (d, *J* = 15.5 Hz, 1H), 7.62 - 7.55 (m, 3H), 7.43 - 7.38 (m, 3H), 6.97 (d, *J* = 2.8 Hz, 1H), 6.88 (dd, *J* = 9.0, 2.8 Hz, 1H), 6.59 (d, *J* = 15.5 Hz, 1H), 3.81 (s, 3H).

To a suspension of (2E)-*N*-(2-chloro-4-methoxyphenyl)-3-phenylprop-2-enamide (**4**) (3.6 g, 12.5 mmoles, 1 eq.) in chlorobenzene (11.2 mL) was added aluminium trichloride (10 g, 75.1 mmoles, 6 eq.). The resulting reaction mixture was stirred at 130°C for 4 hours under an inert atmosphere of argon. The reaction mixture was cooled to room temperature and poured onto an ice/water mix. The resulting precipitate was filtered, rinsed with water and dried under reduced pressure in a desiccator to afford 8-chloro-6-hydroxy-1,2-dihydroquinolin-2-one (**5**) (1.4 g, 57%).
¹H NMR (300 MHz, *d6*-DMSO) δ 10.79 (s, 1H), 9.86 (s, 1H), 7.84 (d, *J* = 9.5 Hz, 1H), 7.13 (d, *J* = 2.5 Hz, 1H), 7.02 (d, *J* = 2.5 Hz, 1H), 6.54 (d, *J* = 9.5 Hz, 1H).

A reaction mixture of 8-chloro-6-hydroxy-1,2-dihydroquinolin-2-one (**5**) (2 g, 10.2 mmoles, 1 eq.) in POCl₃ (9.5 mL) was stirred at 110°C for 4 hours. The reaction mixture was cooled to room temperature then water was slowly added. The resulting precipitate was filtered, rinsed with water and dried under reduced pressure in a desiccator to afford 2,8-dichloroquinolin-6-ol (**6**) (1.6 g, 73%).
¹H NMR (300 MHz, *d6*-DMSO) δ 10.54 (s, 1H), 8.32 (d, *J* = 8.7 Hz, 1H), 7.59 7.52 (m, 2H), 7.23 (d, *J* = 2.6 Hz, 1H).

A reaction mixture of 2,8-dichloroquinolin-6-ol (**6**) (1 g, 4.7 mmoles, 1 eq.), 4-(2-chloroethyl)morpholine hydrochloride (**7**) (869 mg, 4.7 mmoles, 1 eq.), KI (775 mg, 4.7 mmoles, 1 eq.), cesium carbonate (4.5 g, 14.0 mmoles, 1 eq.) in anhydrous DMF (9.3 mL) was stirred at 90°C for 20 hours under an inert atmosphere of argon. The reaction mixture was then concentrated under reduced pressure and the resulting residue was diluted with ethyl acetate. The organic phase was washed with a saturated aqueous solution of brine, dried over MgSO₄, filtered and concentrated under reduced pressure. The resulting residue was purified by column chromatography on silica gel to afford 2,8-dichloro-6-(2-morpholinoethoxy)quinoline (**8**) (930 mg, 61%).
¹H NMR (300 MHz, CDCl₃) δ 8.00 (d, *J* = 8.6 Hz, 1H), 7.57 (d, *J* = 2.7 Hz, 1H), 7.41 (d, *J* = 8.6 Hz, 1H), 7.03 (d, *J* = 2.7 Hz, 1H), 4.22 (t, *J* = 5.6 Hz, 2H), 3.76 (t, *J* = 4.8 Hz, 4H), 2.87 (t, *J* = 5.6 Hz, 2H), 2.61 (t, *J* = 4.8 Hz, 4H).

A reaction mixture of 2,8-dichloro-6-(2-morpholinoethoxy)quinoline (**8**) (164 mg, 0.5 mmol, 1 eq.), 2-ammo-4-trifluoromethylpyridine (**9**) (81 mg, 0.5 mmol, 1 eq.), Pd(OAc)₂ (2.2 mg, 0.01 mmol, 2 mol%), XantPhos (5.8 mg, 0.01 mmol, 2 mol%) and Cs₂CO₃ (456 mg, 1.4 mmol, 2.8 eq.) in *t*-BuOH (2 mL) was heated in a microwave reactor at 120°C for 30 minutes. Upon cooling to room temperature, the reaction mixture was concentrated under reduced pressure and the resulting residue was diluted with ethyl acetate. The organic phase was then washed with water, dried over MgSO₄, filtered and concentrated under reduced pressure. The resulting residue was purified by column chromatography on silica gel to afford 8-chloro-6-(2-morpholinoethoxy)-*N*-(4-(trifluoromethyl)pyridin-2-yl)quinolin-2-amine (1) (117 mg, 52%).
¹H NMR (300 MHz, CDCl₃) δ 9.48 (s, 1H), 8.42 (d, *J* = 5.0 Hz, 1H), 8.00 7.88 (m, 2H), 7.52 (d, *J* = 2.2 Hz, 1H), 7.16 (d, *J* = 5.0 Hz, 1H), 7.07 - 6.96 (m, 2H), 4.21 (t, *J* = 5.5 Hz, 2H), 3.77 (t, *J* = 4.2 Hz, 4H), 2.87 (t, *J* = 5.5 Hz, 2H), 2.62 (t, *J* = 4.2 Hz, 4H).
MS (ESI) [M+H]⁺ = 453.2

### Example 2: Modulation of miR-124 expression by compound of formula (1) on an inflammatory bowel disease in vivo model

### A. Material & Methods

### Ex vivo studies

### Extraction of PBMC using a FICOLL™ gradient

For that purpose, Peripheral blood mononuclear cells (PBMCs) of several healthy donors have been isolated by centrifugation on a FICOLL™ gradient according to standard protocols.

Briefly, 60-70 mL of buffy-coat are poured in a flask of 175 cm², and the volume is adjusted to 300 mL using PBS in order to obtain a dilution of about 5-fold of the buffy coat. 38 mL of diluted Buffy are then added to Falcon™ tubes of 50 mL comprising 12 mL of FICOLL™ (Histopack-1077) at ambient temperature. The preparation is centrifugated for 30 minutes at 515 rcf at ambient temperature. The lymphocyte ring is recovered from the Falcon™ tube with a transfer pipette (Pastette®) and then washed with PBS using centrifugation for 10 minutes at 290 rcf and at ambient temperature until the supernatant becomes clear.

The cells are then resuspended at 37°C to a density of 1,5x10⁶ cells/mL in RPMI Glutamax medium (Life Technologies Ref 61870-010) supplemented with 10% fetal calf serum (FCS) (Thermo Fischer Ref SV30160.03) and without activation. Cells are incubated for 48 hours at 37°C under 5% CO₂.

### Treatment of cells with screened molecules

Six-well plates are used for the screening. Within each well comprising 3.10⁶ cell/4 ml RPMI supplemented with 10% fetal calf serum and 40 U/mL IL-2 (Peprotech Ref 200-02) are added screened molecules. 100% DMSO (4 µL) is added to the well and tested as a negative control.

Each tested condition is set up as described here below and the final corresponding volume is adjusted accordingly in the well:
1) Quinoline derivative in 100% DMSO - (5 µM and final volume 4µL)
2) Antiretroviral drugs: Maraviroc, Efavirenz, Darunavir, AZT (10 µM for all - final volume 4µL).

The wells are incubated for three days at 37°C under 5% CO₂. Medium is changed (Day 3) according to standard protocols. Briefly, plates are centrifugated at 290 rcf for 5 minutes and 3 mL of supernatant is removed. 3 mL of RPMI supplemented with 10% fetal calf serum and 40 U/mL IL-2 is then added with 3 µL of a stock solution of screened molecule at 5 mM in 100% DMSO or 3 µL of 100% DMSO as a negative control.

### Extraction of miRNAs (Day 6)

Cells are recovered within Falcon™ tubes of 15 mL, centrifugated at 290 rcf for 5 minutes, and then washed in 10 mL PBS and further centrifugated at 290 rcf for 5 minutes. Cells are then resuspended in 1 mL PBS and counted.

6x10⁶ cells are recovered and centrifugated at 290 rcf for 5 minutes. The cell pellet is lysed in 300 µL of ML lysis buffer from the Macherey Nagel Nucleospin® miRNA extraction kit ***(Macherey Nagel Ref 740971)*,** and further stored at -20°C.

5 µL of 2x10⁸ copies /µL of spike-in control (Ce_miR-39 from QIAGEN© - reference 219610 of **SEQ ID N°6)** are added for each sample. The miRNA extraction is achieved using the protocol from Macherey Nagel Nucleospin® miRNA extraction kit using an elution volume for RNAs of 50 µL and miRNAs of 30 µL, and further stored at - 20°C.

### Reverse transcription of miRNAs (Day 6)

The reverse transcription step is followed for 12 µL of miRNA using the miScript RT II reverse transcription (RT) kit from QIAGEN© using the miScript HiSpec buffer, and further stored at -20°C.

### Quantitative PCR of miRNAs (Day 6)

The quantitative PCR step is achieved using the QIAGEN© miScript SYBR® Green PCR kit and miScript Primer Assays according to the manufacturer's protocol.

**Composition of the miScript reaction mix for 384-well plates:**

| Mix | µL/reaction |
|---|---|
| 2X SYBR® Green mix | **5** |
| 10X Universal Primer | 1 |
| 10X Primer Assay | 1 |
| H₂0 | 2 |
| Total Mix volume: | **9** |
| Template cDNA in H₂O (*) | **1** |
| Final volume: | **10** |

| | |
|---|---|
| (*) cDNA prepared using the miScript II RT kit | |

The reaction is repeated in triplicates in a 384-well plate according to the manufacturer's protocol on a LightCycler® 380 Roche Real-Time PCR system. Cycling conditions are also set up according to the manufacter's protocol:

| Step | Time | Temperature |
|---|---|---|
| Initial activation step 3-step cycling: | 15 min | 95°C |
| Denaturation | 15 s | 94°C |
| Annealing | 30 s | 55°C |
| Extension | 30 s | 70°C |
| Cycle number | 40 cycles | |

Relative and Absolute quantification of qPCR are known techniques in the Art and can be achieved as further detailed below.

### Relative quantification

From a dilution to the 1/10^{th} in H₂O for the miR-124 qPCR (Hs_miR-124a) or to the 1/100^{th} for reference/housekeeping gene qPCR (Hs_miR-26a and Hs_miR-191, using miScript Primer Assays (Hs_miR-124a, Hs_miR-26a and Hs_miR-191 or QIAGEN© - references ms00006622, ms00029239 and ms00003682).

The analysis is achieved using relative quantification models without efficiency correction (2^{-ΔΔCp}) using the average of crossing points (Cp) values from triplicates of miR-124 and the average of the average of triplicates of miR-26a and miR-191.

### B. Results

One set of donors (4 to 5 donors) has been evaluated. Using the protocol described above the mean fold change (in comparison to DMSO) in miR-124 expression was assessed with different sets of donors (either 4 or 5) by relative quantification and is presented in the Table 1 herebelow:

**Table 1**

| **Molecule** | **Fold-change compared to DMSO treated cells** | | **Number of donors tested** |
|---|---|---|---|
| | **Mean** | **SD** | |
| **Compound of formula (1)** | 32.04 | 55.09 | 5 |
| **Maraviroc** | 0.73 | 0.56 | 4 |
| **Effavirenz** | 0.46 | 0.27 | 4 |
| **Darunavir** | 1.08 | 0.89 | 4 |
| **AZT** | 0.90 | 0.55 | 4 |

Thus, experimental evidence shows that said compound induced significant up-regulation of miR-124. In contrast none of the known antiretroviral (Maraviroc, Effavirenz, Darunavir or AZT) has any significant effect on the overexpression of miR-124 in PBMCs from four donors.

### Example 3: Inhibition of HIV-1 production in infected peripheral blood mononuclear cells (PBMCs)

### MATERIAL AND METHODS

The first determination is that of the concentration of compound that exhibits the fewest side effects in terms of cell viability and progression of the cell cycle.

Within this framework, the peripheral blood mononuclear cells (PBMCs) of healthy donors are isolated by centrifugation on a FICOLL gradient. The cells are then activated two days to a density of 1.5 x 10⁶ cells/ml in RPMI plutamax medium supplemented with 10% fetal calf serum (FCS), 40 U/ml of IL2 and 5 µg/ml PHA, in an incubator at 37 °C, 5% CO₂.

A standard experiment using 96 plates to test 30 molecules in triplicates including positive and negative controls, is performed as follows:
PHA/IL2 activated PBMCs are washed with RPMI 10% FCS and resuspended at 1.5 x 10⁶ cells/ml in RPMI glutamax 10% FCS, 40U/ml Il2. The cells are seeded in 96 wells (1.5 10⁵ cells/well/100µl). Viral infection is performed with 1 ng of AdaM/well. 100 µl of tested molecules at concentration of 20 µM are added to each well (10µM final concentration). Virus production is determined by p24 antigen immunosorbent assays after 3 and 6 days of infection (Kit Innogenetics). Typically PBMCs are prepared from several healthy donors (around 11 different donors). Dose response curves were then established with selected compounds to determine IC₅₀.

### Protocol for cytotoxicity:

To evaluate the cytoxicity of different compounds we used the same protocol as above to seed PBMCs in a final volume of 50 µl, without adding the virus, and 50µl of tested molecules. After an incubation for 6 days at 37°C, 20µl of CellTiter96 AqueousOne solution is added to determine the number of viable cells in proliferation and cytotoxicity assays (Promega). CellTiter96 AqueousOne is a colorimetric assay solution that has many advantages compared to MTT assays and gives us satisfactory results.

### Results:

The efficacy of compound of formula (1) and of compound n°1 as disclosed in document WO 2012/080953 is measured by the HIV-specific enzyme-linked immunosorbent assay, p24 ELISA.

Said compound n°1 as disclosed in document WO 2012/080953 presents the following structure:

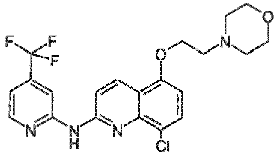

Drug efficacy is expressed as percent inhibition of the HIV p24 antigen in this rapid and sensitive assay. It is expected that compounds of the present invention exhibit an IC₅₀ of less than 10 µM, or even less than 1 µM *in vitro.*

The following results may be reported (mean of 4 donors) in table 2 as follows:

**Table 2**

| Number of the tested compound | Activity (IC₅₀) |
|---|---|
| Compound of formula (1) | 0.43 |
| Compound n°1 as disclosed in document WO2012/080953 | 62.31 |

Thus, experimental evidence shows that the compound of formula (1) up-regulate the expression levels of miR-124 in PBMCs. Therefore, the result of the tests carried out on the compound of formula (1) disclosed in the present invention show that said compound of formula (1) may be useful to treat and/or prevent inflammatory diseases as described further above.

Experimental evidence further establishes that the compound of formula (1) show that it is relevant as active substance in inhibiting or treating AIDS, an AIDS-related condition and/or HIV.

For this purpose an effective amount of a said compound may be administered to an individual suffering from inflammatory diseases or AIDS, an AIDS-related condition and/or HIV.

Thus, the compound according to the present invention may be implemented within pharmaceutical composition that may contain an effective amount of said compound, and one or more pharmaceutical excipients.

The aforementioned excipients are selected according to the dosage form and the desired mode of administration.

In this context they can be present in any pharmaceutical form which is suitable for enteral or parenteral administration, in association with appropriate excipients, for example in the form of plain or coated tablets, hard gelatine, soft shell capsules and other capsules, suppositories, or drinkable, such as suspensions, syrups, or injectable solutions or suspensions, in doses which enable the daily administration of from 0.1 to 1000 mg of active substance.

Any route of administration may be used. For example, the compound of formula (1) can be administered by oral, parenteral, intravenous, transdermal, intramuscular, rectal, sublingual, mucosal, nasal, or other means. In addition, the compound of formula (1) can be administered in a form of pharmaceutical composition and/or unit dosage form.

In particular, pharmaceutical compositions of the invention may be administered orally and/or parenterally.

### SEQUENCE LISTING

**SEQ ID N°1**
**SEQ ID N°2**
**SEQ ID N°3**
**SEQ ID N°4**
   UAAGGCACGCGGUGAAUGCC
**SEQ ID N°5**
   CGUGUUCACAGCGGACCUUGAU
**SEQ ID N°6**
   UCACCGGGUGUAAAUCAGCUUG

## Claims

1. Compound of formula (1) 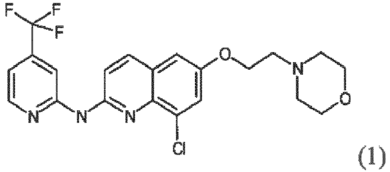 in the form of a base or addition salt with an acid, particularly a pharmaceutically acceptable acid.

2. Compound according to claim 1, for use as a medicament.

3. Compound of formula (1) according to claim 1 for use in the treatment of retroviral infection and in particular AIDS or an AIDS-related condition or Human Immunodeficiency virus (HIV).

4. Compound of formula (1) according to claim 1 for use in the treatment and/or prevention of inflammatory disease.

5. Compound of formula (1) for use according to the preceding claim, wherein said inflammatory disease is selected in the list consisting of: an inflammatory disease associated with an autoimmune disease, a central nervous system (CNS) inflammatory disease, a joint inflammation disease, an inflammatory digestive tract disease, inflammatory skin and other inflammatory diseases related to epithelial cells, and inflammation associated with cancer, inflammation associated with irritation, and inflammation associated with injury.

6. Compound of formula (1) for use according to the preceding claim, wherein said inflammatory disease is selected in the list consisting of: Inflammatory Bowel Disease, Rheumatoid Arthritis, Crohn's disease, Ulcerative Colitis, Multiple Sclerosis, osteoarthritis, ankylosing spondylitis, psoriasis, Sjogren's syndrom, bronchitis, and colon carcinoma.

7. Pharmaceutical composition comprising the compound according to Claim 1 and at least one pharmaceutically acceptable excipient.

8. Medicament comprising the compound according to Claim 1.

9. Process for preparing a compound according to Claim 1, comprising: the step of reacting a compound of formula (8) 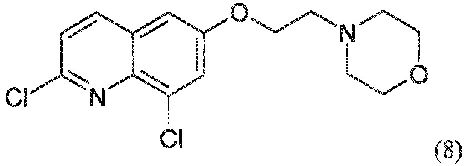 with a compound of formula (9) 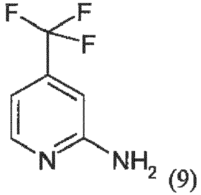 in the presence of an inorganic base such as a carbonate base, for example cesium, potassium or sodium, or a tert-butoxide base, for example cesium, potassium or sodium, in the presence of a catalyst, preferably a Palladium catalyst, and preferably in the presence of a ligand such as a phosphine ligand or a ferrocene-derived ligand, for example a bidentate phosphine ligand, optionally with heating at a temperature ranging from 90 to 150°C.

10. Compound of formula (8)

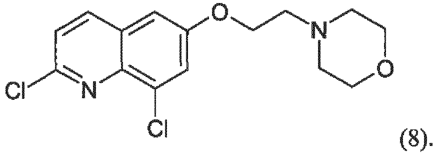

## Patentansprüche

1. Verbindung nach Formel (1) 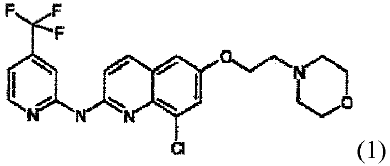 in der Form einer Base oder eines Additionssalzes mit einer Säure, insbesondere einer pharmazeutisch verträglichen Säure.

2. Verbindung gemäß Anspruch 1 zur Verwendung als ein Medikament.

3. Verbindung nach Formel (1) gemäß Anspruch 1 zur Verwendung in der Behandlung von retroviraler Infektion und insbesondere AIDS oder einem mit AIDS in Zusammenhang stehendem Zustand oder Humane Immundefizienz-Virus (HIV).

4. Verbindung nach Formel (1) gemäß Anspruch 1 zur Verwendung in der Behandlung und/oder Vorbeugung einer Entzündungskrankheit.

5. Verbindung nach Formel (1) zur Verwendung gemäß dem vorhergehenden Anspruch, wobei die Entzündungskrankheit aus der Liste ausgewählt ist, die besteht aus: einer Entzündungskrankheit in Zusammenhang mit einer Autoimmunerkrankung, einer entzündlichen Zentralnervensystem (ZNS)-Erkrankung, einer entzündlichen Gelenkerkrankung, einer entzündlichen Verdauungstrakterkrankung, Hautentzündung und weiteren Entzündungskrankheiten in Zusammenhang mit Epithelzellen und Entzündung in Zusammenhang mit Krebs, Entzündung in Zusammenhang mit Reizung und Entzündung in Zusammenhang mit Verletzung.

6. Verbindung nach Formel (1) zur Verwendung gemäß dem vorhergehenden Anspruch, wobei die Entzündungskrankheit aus der Liste ausgewählt ist, die besteht aus: entzündlicher Darmerkrankung, rheumatoider Arthritis, Morbus Crohn, Colitis ulcerosa, multipler Sklerose, Osteoarthritis, Morbus Bechterew, Psoriasis, Sjögren-Syndrom, Bronchitis und Kolonkarzinom.

7. Pharmazeutische Zusammensetzung, umfassend die Verbindung gemäß Anspruch 1 und mindestens einen pharmazeutisch verträglichen Exzipienten.

8. Medikament, umfassend die Verbindung gemäß Anspruch 1.

9. Verfahren zur Herstellung einer Verbindung gemäß Anspruch 1, umfassend: den Schritt von Umsetzen einer Verbindung nach Formel (8) 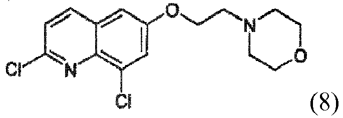 mit einer Verbindung nach Formel (9) 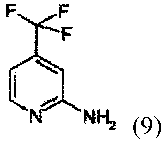 in der Gegenwart einer anorganischen Base wie einer Carbonat-Base, zum Beispiel Cäsium, Kalium oder Natrium, oder einer tert-Butoxid-Base, zum Beispiel Cäsium, Kalium oder Natrium, in der Gegenwart eines Katalysators, bevorzugt eines Palladium-Katalysators, und bevorzugt in der Gegenwart eines Liganden wie eines Phosphin-Liganden oder eines von Ferrocen abgeleiteten Liganden, zum Beispiel eines zweizähnigen Phosphin-Liganden, gegebenenfalls mit Erwärmen bei einer Temperatur in einem Bereich von 90 bis 150°C.

10. Verbindung nach Formel (8)

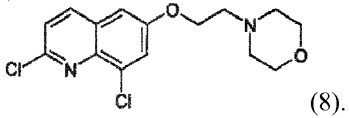

## Revendications

1. Composé de formule (1) 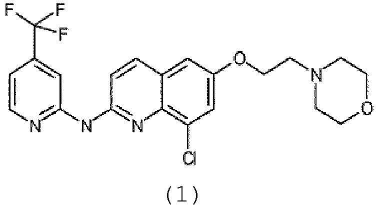 sous la forme d'une base ou d'un sel d'addition avec un acide, en particulier un acide pharmaceutiquement acceptable.

2. Composé selon la revendication 1, pour une utilisation en tant que médicament.

3. Composé de formule (1) selon la revendication 1, pour une utilisation dans le traitement d'une infection rétrovirale et en particulier du SIDA ou d'un état lié au SIDA ou par le virus de l'immunodéficience humaine (VIH).

4. Composé de formule (1) selon la revendication 1, pour une utilisation dans le traitement et/ou la prévention d'une maladie inflammatoire.

5. Composé de formule (1) pour une utilisation selon la revendication précédente, dans lequel ladite maladie inflammatoire est choisie dans la liste constituée par : une maladie inflammatoire associée à une maladie auto-immune, une maladie inflammatoire du système nerveux central (SNC), une maladie inflammatoire articulaire, une maladie inflammatoire des voies digestives, une maladie inflammatoire de la peau et d'autres maladies inflammatoires associées à des cellules épithéliales, et une inflammation associée à un cancer, une inflammation associée à une irritation, et une inflammation associée à une blessure.

6. Composé de formule (1) pour une utilisation selon la revendication précédente, dans lequel ladite maladie inflammatoire est choisie dans la liste constituée par : une maladie intestinale inflammatoire, la polyarthrite rhumatoïde, la maladie de Crohn, la rectocolite hémorragique, la sclérose en plaques, l'arthrose, la spondylarthrite ankylosante, le psoriasis, le syndrome de Sjogren, la bronchite, et le carcinome du côlon.

7. Composition pharmaceutique comprenant le composé selon la revendication 1 et au moins un excipient pharmaceutiquement acceptable.

8. Médicament comprenant le composé selon la revendication 1.

9. Procédé pour préparer un composé selon la revendication 1, comprenant : une étape de réaction d'un composé de formule (8) 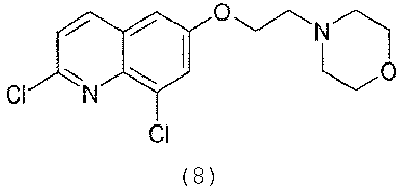 avec un composé de formule (9) 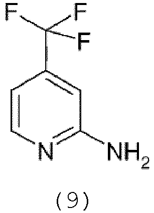 en présence d'une base inorganique telle qu'une base de type carbonate, par exemple de césium, potassium ou sodium, ou une base de type tert-butylate, par exemple de césium, de potassium ou de sodium, en présence d'un catalyseur, de préférence un catalyseur au palladium, et de préférence en présence d'un ligand tel qu'un ligand de type phosphine ou un ligand dérivé de ferrocène, par exemple un ligand de type phosphine bidenté, éventuellement avec chauffage à une température située dans la plage allant de 90 à 150°C.

10. Composé de formule (8)


### SEQUENCE LISTING

<110> ABIVAX
   CENTRE NATIONAL DE LA RECHERCHE SCIENTIFIQUE
   INSTITUT CURIE
   UNIVERSITE DE MONTPELLIER
<120> A QUINOLINE DERIVATIVE FOR THE TREATMENT OF INFLAMMATORY DISEASES AND AIDS
<130> PR73255
<150> EP14306166.1
   <151> 2014-07-17
<160> 6
<170> BiSSAP 1.3
<210> 1
   <211> 85
   <212> RNA
   <213> Homo sapiens
<400> 1
   aggccucucu cuccguguuc acagcggacc uugauuuaaa uguccauaca auuaaggcac 60
   gcggugaaug ccaagaaugg ggcug 85
<210> 2
   <211> 109
   <212> RNA
   <213> Homo sapiens
<400> 2
   aucaagauua gaggcucugc ucuccguguu cacagcggac cuugauuuaa ugucauacaa 60
   uuaaggcacg cggugaaugc caagagcgga gccuacggcu gcacuugaa 109
<210> 3
   <211> 87
   <212> RNA
   <213> Homo sapiens
<400> 3
   ugagggcccc ucugcguguu cacagcggac cuugauuuaa ugucuauaca auuaaggcac 60
   gcggugaaug ccaagagagg cgccucc 87
<210> 4
   <211> 20
   <212> RNA
   <213> Homo sapiens
<400> 4
   uaaggcacgc ggugaaugcc 20
<210> 5
   <211> 22
   <212> RNA
   <213> Homo sapiens
<400> 5
   cguguucaca gcggaccuug au 22
<210> 6
   <211> 22
   <212> RNA
   <213> Caenorhabditis elegans
<400> 6
   ucaccgggug uaaaucagcu ug 22